(19) Europäisches Patentamt
European Patent Office
Office européen des brevets

(11) **EP 2 000 080 B1**

(12) **EUROPEAN PATENT SPECIFICATION**

(45) Date of publication and mention
of the grant of the patent:
**17.02.2010 Bulletin 2010/07**

(51) Int Cl.:
***A61B 3/103*** *(2006.01)*     ***A61B 3/107*** *(2006.01)*

(21) Application number: **08157442.8**

(22) Date of filing: **02.06.2008**

(54) **Ophthalmic apparatus and a method to determine power of an intraocular lens**

Ophthalmische Vorrichtung und Verfahren zur Bestimmung der Kraft einer intraokularen Linse

Appareil ophtalmique et procédé pour déterminer la puissance d'une lentille intraoculaire

(84) Designated Contracting States:
**DE FR GB**

(30) Priority: **04.06.2007 JP 2007148688**

(43) Date of publication of application:
**10.12.2008 Bulletin 2008/50**

(73) Proprietor: **Nidek Co., Ltd.**
**Gamagori-shi**
**Aichi 443-0038 (JP)**

(72) Inventors:
• **Shimizu, Kazunari**
**Gamagori-shi 443-0120 (JP)**
• **Ban, Yukinobu**
**Nishio-shi Aichi 445-0874 (JP)**
• **Kamikawa, Tomohiro**
**Okazaki-shi 444-0806 (JP)**

(74) Representative: **Winter, Brandl, Fürniss, Hübner**
**Röss, Kaiser,**
**Polte Partnerschaft Patent- und**
**Rechtsanwaltskanzlei**
**Alois-Steinecker-Strasse 22**
**85354 Freising (DE)**

(56) References cited:
**WO-A-2005/030044**     **US-A1- 2005 225 724**

• **ROSA N ET AL: "A NEW METHOD OF CALCULATING INTRAOCULAR LENS POWER AFTER PHOTOREFRACTIVE KERATECTOMY" JOURNAL OF REFRACTIVE SURGERY, THOROFARE, NJ, US, vol. 18, no. 6, 1 November 2002 (2002-11-01), pages 720-724, XP008040519 ISSN: 1081-597X**

**Description**

BACKGROUND OF THE INVENTION

1. Field of the Invention

This invention relates to an ophthalmic apparatus which obtains characteristics of a cornea of an examinee's eye which are used to determine (prescribe) power of an intraocular lens to be injected into the eye, and a method to determine the intraocular lens power using the corneal characteristics.

2. Description of Related Art

In cataract surgery, corneal refractive power (distribution of corneal surface refractive power) and an ocular axial length of an examinee's eye are used to determine power of an intraocular lens to be injected into the eye (a lens capsule) after a lens nucleus is removed (see "Corneal topographer and wavefront sensor" by Naoyuki Maeda et al., published on October 10, 2002 by Medical View Co., Ltd.).

To obtain the corneal refractive power, a corneal shape measurement apparatus (e.g. a keratometer) is often used which measures a corneal shape (distribution of corneal curvature radius) by picking up an image of a measurement target projected onto the cornea. The corneal shape measurement apparatus measures a shape (distribution of curvature radius) of a corneal anterior surface and does not measure a shape (distribution of curvature radius) of a corneal posterior surface. Hence, with the assumption that the ratio between the curvature radius distribution of the corneal anterior surface and the curvature radius distribution of the corneal posterior surface is uniform regardless of differences in examinees' eyes, a correction refractive index (n=1.3375 in general) which is referred to as the Keratometric index is used to obtain the corneal refractive power.

However, when determining power of an intraocular lens to be injected into an examinee's eye which has undergone refractive surgery, desired post-operative visual acuity often cannot be obtained (a hyperopic shift of 1D to 3D (D=Diopter) often occurs) if the intraocular lens power is determined using the corneal refractive power obtained by applying the above correction refractive index to the eye which has undergone the refractive surgery. This is because that the ratio between the curvature radius distributions of the corneal anterior surface and the corneal posterior surface after the refractive surgery differs from the ratio assumed as above.

Document WO-A-2005/030044 relates to a method for measuring an optimally adapted intraocular lens for patients having a refractively modified cornea.

SUMMARY OF THE INVENTION

An object of the invention is to provide an ophthalmic apparatus capable of obtaining characteristics of a cornea which are suitable for determining (prescribing) power of an intraocular lens to be injected into an examinee's eye which has undergone refractive surgery. Another object of the invention is to provide a method to determine the intraocular lens power using the corneal characteristics, by which desired post-operative visual acuity is obtainable even with the eye which has undergone the refractive surgery.

To achieve the objects and in accordance with the purpose of the present invention, an ophthalmic apparatus which obtains characteristics of a cornea of an examinee's eye which are used to determine power of an intraocular lens to be injected into the eye comprises an input unit which inputs data on a shape of the cornea after refractive surgery, and a calculation unit having a program which calculates post-operative corneal refractive power based on the post-operative corneal shape, wherein the program determines a non-corrected region of the cornea based on the post-operative corneal shape, estimates a pre-operative corneal shape in a corrected region by calculating an approximate curve from a corneal shape in the non-corrected region, calculates pre-operative corneal refractive power based on the pre-operative corneal shape, calculates correction refractive power in the refractive surgery based on the post-operative corneal shape and the pre-operative corneal shape, and calculates post-operative corneal refractive power based on the pre-operative corneal refractive power and the correction refractive power.

In another embodiment of the present invention, a method to determine power of an intraocular lens to be injected into an examinee's eye comprises the steps of inputting data on a shape of a cornea after refractive surgery, determining a non-corrected region of the cornea based on the post-operative corneal shape, estimating a pre-operative corneal shape in a corrected region by calculating an approximate curve from a corneal shape in the non-corrected region, calculating pre-operative corneal refractive power based on the pre-operative corneal shape, calculating correction refractive power in the refractive surgery based on the post-operative corneal shape and the pre-operative corneal shape, and calculating post-operative corneal refractive power based on the pre-operative corneal refractive power and the correction refractive power, and determining the intraocular lens power using the post-operative corneal refractive power and an ocular axial length of the eye.

Additional objects and advantages of the invention are set forth in the description which follows, are obvious from the description, or may be learned by practicing the invention. The objects and advantages of the invention may be realized and attained by the apparatus in the claims.

BRIEF DESCRIPTION OF THE DRAWINGS

The accompanying drawings, which are incorporated in and constitute a part of this specification, illustrate embodiments of the present invention and, together with the description, serve to explain the obj ects, advantages and principles of the invention. In the drawings,

Fig. 1 is a view showing a schematic configuration of an ophthalmic apparatus according to a preferred embodiment of the present invention;
Fig. 2 is a flowchart showing calculation of corneal refractive power; and
Fig. 3 is a schematic sectional view of a plane cutting through a three-dimensional shape of the cornea in parallel with a measurement optical axis which passes through the corneal vertex.

DETAILED DESCRIPTION OF THE PREFERRED EMBODIMENTS

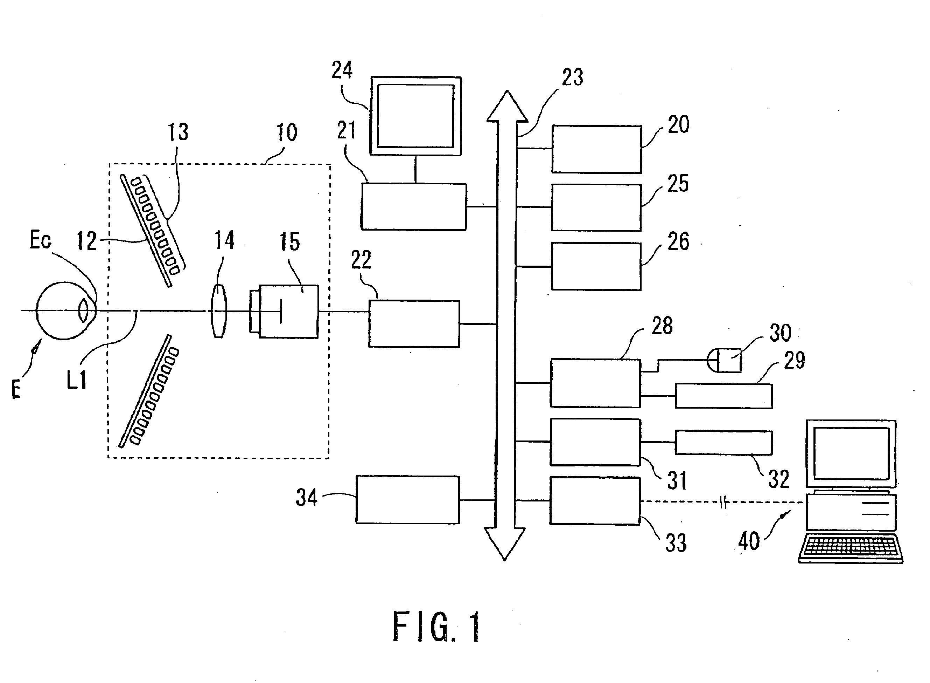

A detailed description of one preferred embodiment of an ophthalmic apparatus embodied by the present invention is provided below with reference to the accompanying drawings. Fig.1 is a view showing a schematic configuration of an ophthalmic apparatus according to a preferred embodiment of the present invention. The apparatus obtains characteristics of a cornea of an examinee's eye which are used to determine power of an intraocular lens to be injected into the eye.

A measurement optical system 10 which measures a shape (a corneal shape) and refract ive power (corneal refractive power) of a cornea Ec of an examinee's eye E by picking up an image of a measurement target projected onto the cornea Ec comprises a placido ring plate 12 for projecting a ring-pattern target defining the measurement target onto the cornea Ec, an illumination light source 13, an image-pickup lens 14 and a CCD camera 15 for picking up the image of the ring-pattern target projected onto the cornea Ec. The lens 14 and the camera 15 also serve as an observation optical system for observing an anterior segment of the eye E. In addition to the above optical systems, the apparatus comprises systems (not shown) such as a fixation target presenting optical system and an alignment condition detecting optical system; however, detailed explanation on these systems are omitted here because known configurations of an ophthalmic apparatus can be used.

The ring-pattern target image picked up by the camera 15 is captured by a video capture 22 connected to a calculation and control unit (a CPU) 20 via a bus 23. Based on the ring-pattern target image picked up by the camera 15, the calculation and control unit 20 performs processes such as measurement (calculation) of the corneal shape (distribution of corneal curvature radius) and measurement (calculation) of the corneal refractive power (distribution of corneal surface refractive power). In addition, the calculation and control unit 20 controls operations of the apparatus. An image processing unit 21 connected to the calculation and control unit 20 via the bus 23 is also connected to a liquid crystal display (a monitor) 24 capable of color display and controls display of data such as images and measurement results on the display 24. The bus 23 is connected with a memory 25 which stores data such as images and measurement results, a hard disk (an HDD) 26 which stores data such as a program, a serial I/O 28 connected with a keyboard 29 and a mouse 30, a parallel I/O 31 connected with a printer 32, a communication port 33, and an operation unit (a switch unit) 34 having various switches. The communication port 33 is connectable to an external computer 40 and capable of transmitting and receiving data. Accordingly, the computer 40 may have functions of the video capture 22 and the components thereafter.

The picking up of the ring-pattern target image and the measurements (the calculations) thereafter are described as follows.

When alignment is made between the eye E and the measurement optical system 10 and an image-pickup switch of the operation unit 34 is pressed, the calculation and control unit 20 controls to light the light source 13 and picks up the ring-pattern target image with the camera 15. The picked-up ring-pattern target image is stored in the memory 25. The calculation and control unit 20 detects edges of rings in the ring-pattern target image stored in the memory 25, sends the result to the image processing unit 21, and displays it on the display 24.

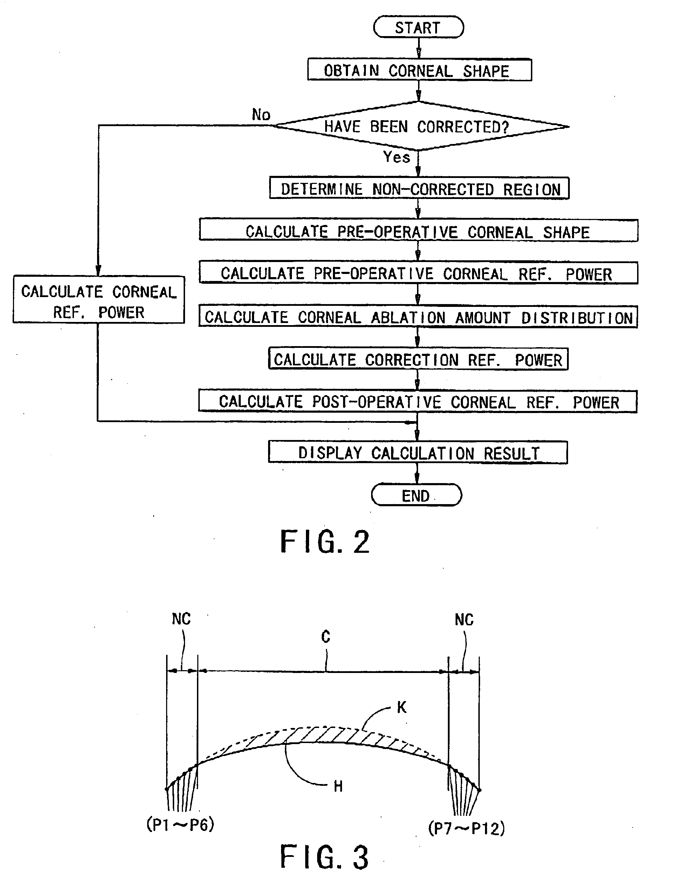

Fig. 2 is a flowchart showing the calculation of the corneal refractive power based on the obtained ring-pattern target image. The calculation and control unit 20 obtains the corneal shape based on the ring-pattern target image by executing a corneal shape measurement program. Then, the calculation and control unit 20 calculates the corneal refractive power based on the obtained corneal shape by executing a corneal refractive power measurement program. The measurement programs are executed by operations such as clicking on a measurement key displayed on the display 24 with the mouse 30.

Before measuring the corneal refractive power, the calculation and control unit 20 determines whether or not the cornea Ec has undergone refractive surgery based on the corneal shape in the vicinity of the corneal center (see USP 2005/0225724A corresponding to Japanese Patent Application Unexamined Publication No. 2005-288176).

If it is determined that the cornea Ec has not undergone the refractive surgery, the calculation and control unit 20 calculates the corneal refractive power based on the obtained corneal shape, displays the result on the display 24, and stores it in the memory 25.

When obtaining the corneal shape based on the ring-pattern target image, the calculation and control unit 20 detects the edges of the rings in the ring-pattern target image as mentioned above and obtains the corneal shape at every predetermined angle based on a distance from the corneal center to the edge. Refer to USP 5, 500, 697 B corresponding to Japanese Patent Application Unexamined Publication No. H7-124113 for details regarding how to calculate. For example, if the ring-pattern target has twenty-three ring targets, and meridians which pass through the corneal center and are provided at every one degree are used as measurement meridians, 23x360 corneal shapes are obtainable centering the corneal center as the spherical coordinate center.

Then, the calculation and control unit 20 converts the obtained corneal shape (the obtained corneal curvature radius distribution) within a predetermined measurement region into the corneal refractive power (the corneal surface refractive power distribution). Here, Formula (1) for calculating the corneal surface refractive power only from a curvature radius of the corneal anterior surface ($r_A$) is used, and, with the assumption that the ratio between the curvature radius distributions of the corneal anterior surface and the corneal posterior surface is uniform, a correction refractive index (n=1.3375) is used.

$$\text{Formula 1:} \quad P(D) = (1.335 - 1.0000)/r_A \times 10^{-3} \quad (D = \text{Diopter})$$

Refer to USP 6, 033, 075 B corresponding to Japanese Patent Application Unexamined Publication No. H11-342152 for details regarding how to convert the obtained corneal shape (the obtained corneal curvature radius distribution) into the corneal refractive power (the corneal surface refractive power distribution).

If it is determined that the cornea Ec has undergone the refractive surgery, the calculation and control unit 20 determines a non-corrected region after the refractive surgery based on the obtained post-operative corneal shape in the predetermined measurement region. To determine the non-corrected region, the calculation and control unit 20 divides the obtained post-operative corneal shape into a corneal shape in the non-corrected region and a corneal shape in a corrected region. More specifically, first the obtained post-operative corneal shape (the obtained post-operative corneal curvature radius distribution) in the predetermined measurement region is converted into the post-operative corneal refractive power (the post-operative corneal surface refractive power distribution) . Next, because the corneal refractive powers are relatively high in the vicinity of a boundary position between the corrected region and the non-corrected region, the corneal refractive powers at corneal positions from the corneal center region to the corneal peripheral region are monitored. Then, positions in which amounts of change in the corneal refractive powers are greater than a predetermined amount of change are detected as boundary positions. A region in the center side (inside) of the boundary positions is determined as the corrected region while a region in the peripheral side of (outside) the boundary positions are determined as the non-corrected region. Alternatively, two-dimensional mapping of the post-operative corneal refractive powers may be displayed and the division between the corrected and non-corrected regions may be performed by operations such as clicking with the mouse 30. Still alternatively, the corrected and non-corrected regions may be divided based on information such as the post-operative corneal shape.

Then, the calculation and control unit 20 calculates (estimates) pre-operative corneal shapes from the obtained post-operative corneal shapes. Fig. 3 is a schematic sectional view of a plane cutting through a three-dimensional shape of the cornea in parallel with a measurement optical axis L1 which passes through the corneal vertex. For an examinee's eye which has undergone myopic correction and whose cornea is ablated by procedures such as irradiation of an excimer laser beam, the corneal center region is determined as a corrected region c and the corneal peripheral region is determined as a non-corrected region NC.

The calculation and control unit 20 calculates the pre-operative corneal shape in the determined corrected region C as an estimated value by calculating an approximate curve from the post-operative corneal shapes in the determined non-corrected region NC. More specifically, at least three points (P1 to P12 in Fig. 3) on post-operative corneal anterior surface curves in the non-corrected area NC are determined, and a spline curve (other manners may be used such as least squares approximation and Q-value distribution of a normal eye) is drawn based on the determined points. Accordingly, the approximate curve (a dotted line K in Fig. 3) of a pre-operative corneal anterior surface curve in the corrected region C is obtained. In the preferred embodiment, the obtained approximate curve in the corrected region C is calculated as the pre-operative corneal shape in the corrected region C. Alternatively, an index indicating an ellipse degree of the cornea (e.g. a Q value) may be used to obtain the approximate curve in the corrected region C. For example, a Q value of the cornea may be determined from the post-operative corneal anterior surface curve in the non-corrected region NC, and the approximate curve in the corrected region C may be obtained using the determined Q value. Still alternatively, mapping of a cross-sectional shape of the cornea may be displayed, and the pre-operative corneal anterior surface curve in the corrected region C may be drawn as a virtual line by operations such as clicking with the mouse 30 using tools such as graphic drawing software, and the approximate curve in the corrected region C may be obtained based on the drawn virtual line.

Then, the calculation and control unit 20 calculates pre-operative corneal refractive power in the corrected region C based on the obtained pre-operative corneal shape in the corrected region C. That is, the obtained pre-operative corneal shape (the obtained pre-operative corneal curvature radius distribution) in the corrected region C is converted into the pre-operative corneal refractive power (the pre-operative corneal refractive power distribution) as described above. Because the obtained pre-operative corneal refractive power is based on the pre-operative corneal shape with which it is possible to assume that the ratio between the curvature radius distributions of the corneal anterior surface and the corneal posterior surface is uniform, a measurement error due to difference in the ratio between the curvature radius distributions of the corneal anterior surface and the corneal posterior surface is avoided.

Next, the calculation and control unit 20 calculates correction (ablation) refractive power in the refractive surgery based on the pre-operative corneal shape and the post-operative corneal shape and then calculates the post-operative corneal refractive power based on the pre-operative corneal refractive power and the correction refractive power. More specifically, distribution of a correction (ablation) amount (H in Fig. 3) in the corrected region C is obtained by calculating a difference between the pre-operative corneal shape and the post-operative corneal shape in the corrected region C, and the correction refractive power is obtained based on the obtained shape of the correction amount distribution. Then, the post-operative corneal refractive power is obtained by calculating a difference between the pre-operative corneal refractive power and the correction refractive power.

After the post-operative corneal refractive power in one meridian direction is obtained as above, the calculation and control unit 20 similarly calculates post-operative corneal refractive powers in other meridian directions. When post-operative corneal refractive powers are obtained at least in three meridian directions, post-operative corneal refractive power with reference to the corneal center is calculated by a least-square method, and the calculation result of the post-operative corneal refractive power is displayed on the monitor 24.

Because the obtained post-operative corneal refractive power is obtained by subtracting the correction refractive power which can be assumed to be the correction amount in the refractive surgery from the pre-operative corneal refractive power with which it is possible to assume that the ratio between the curvature radius distributions of the corneal anterior surface and the corneal posterior surface is uniform, it can be considered that the post-operative corneal refractive power properly presents the current corneal refractive power of the eye which has undergone the refractive surgery. Accordingly, a measurement error of the corneal refractive power due to a difference in the ratio between the curvature radius distributions of the corneal anterior surface and the corneal posterior surface is avoided.

When the intraocular lens power of the eye E is determined (prescribed) using the post-operative corneal refractive power obtained as above, the calculation and control unit 20 determines the intraocular lens power based on the obtained post-operative corneal refractive power and a preinputted ocular axial length and displays it on the monitor 24. As for a formula used to determine the intraocular lens power, known formulas such as SRK II Formula and SRK/T Formula may be used.

If, for example, SRK II Formula is used, the formula is P=A-2.5L-0.9K+C (P: intraocular lens power, A: A constant, L: ocular axial length (mm), K: medium value of corneal curvature radius, C: correction value), and K is calculated by substituting the post-operative corneal refractive power in P of the aforementioned formula (1). If a known Double-K Formula is used, the pre-operative corneal refractive power and the post-operative corneal refractive power are substituted. If formulas such as Hoffer Q Formula and Binkhorst Formula are used which determine the intraocular lens power using as a parameter a distance between a lens surface of the intraocular lens and the cornea when the intraocular lens is injected into the eye, a difference between the distance from the lens surface to the cornea and a correction (ablation) amount at the corneal vertex is substituted along with the post-operative corneal refractive power.

The above procedures allow efficient obtainment of the corneal refractive power suitable for determining the intraocular lens power even for the eye which has undergone the refractive surgery. Accordingly, the eye can be corrected to emmetropia by the inserted intraocular lens.

In the description above, the corneal refractive power of the eye which has undergone myopic correction is measured; however, the present invention is applicable also to eyes such as an eye which has undergone hyperopic correction and an eye which has undergone astigmatic correction.

In addition, in the description above, the pre-operative corneal shape in the corrected region is calculated by determining the non-corrected region based on the obtained post-operative corneal shapes in the predetermined measurement region. However, if the eye (the cornea) which has undergone the refractive surgery has an optical zone which is ablated for correcting the refractive power and a transition zone which is ablated for smoothening the post-operative corneal shape, the pre-operative corneal shape in the corrected region may be calculated by determining a non-corrected region which does not include the optical and transition zones based on the obtained post-operative corneal shape in the predetermined measurement region. In addition, if the non-corrected region is not determined, the pre-operative corneal shape may be calculated based on the post-operative corneal shape in the transition zone.

In the above description, the apparatus which obtains the corneal characteristics and determine the intraocular lens power based on the obtained corneal characteristics is described as an example. However, the present invention is applicable also to an apparatus which determines the intraocular lens power based on the corneal characteristics which are obtained by another apparatus and inputted into the apparatus.

The foregoing description of the preferred embodiments of the invention has been presented for purposes of illustration and description. It is not intended to be exhaustive or to limit the invention to the precise form disclosed, and modifications and variations are possible in the light of the above teachings or may be acquired from practice of the invention. The embodiments chosen and described in order to explain the principles of the invention and its practical application to enable one skilled in the art to utilize the invention in various embodiments and with various modifications as are suited to the particular use contemplated. It is intended that the scope of the invention be defined by the claims appended hereto.

## Claims

1.  An ophthalmic apparatus which obtains characteristics of a cornea of an examinee's eye which are used to determine power of an intraocular lens to be injected into the eye, the apparatus comprising:

    an input unit (20) which inputs data on a shape of the cornea after refractive surgery; and
    a calculation unit (20) having a program which calculates post-operative corneal refractive power based on the post-operative corneal shape, wherein
    the program
    determiners a non-corrected region of the cornea based on the post-operative corneal shape; **characterized in that** the program estimates a pre-operative corneal shape in a corrected region by calculating an approximate curve from a corneal shape in the non-corrected region,
    calculates pre-operative corneal refractive power based on the pre-operative corneal shape;
    calculates correction refractive power in the refractive surgery based on the post-operative corneal shape and the pre-operative corneal shape; and
    calculates post-operative corneal refractive power based on the pre-operative corneal refractive power and the correction refractive power.

2.  The ophthalmic apparatus according to claim 1, wherein the calculation unit further comprises a program which determines the intraocular lens power using the post-operative corneal refractive power and an ocular axial length of the eye.

3.  The ophthalmic apparatus according to claim 1, wherein the program divides the post-operative corneal shape into the corneal shape in the non-corrected region and a corneal shape in the corrected region so as to determine the non-corrected region.

4.  The ophthalmic apparatus according to claim 1 further comprising a corneal shape measurement apparatus (10) which measures the corneal shape by picking up an image of a measurement target projected onto the cornea of the eye, wherein
    the input unit inputs data on the measured post-operative corneal shape.

5.  A method to determine power of an intraocular lens to be injected into an examinee's eye, the method comprising the steps of:

    inputting data on a shape of a cornea after refractive surgery;
    determining a non-corrected region of the cornea based on the post-operative corneal shape;
    estimating a pre-operative corneal shape in a corrected region by calculating an approximate curve from a corneal shape in the non-corrected region;
    calculating pre-operative corneal refractive power based on the pre-operative corneal shape;
    calculating correction refractive power in the refractive surgery based on the post-operative corneal shape and the pre-operative corneal shape; and

calculating post-operative corneal refractive power based on the pre-operative corneal refractive power and the correction refractive power; and

determining the intraocular lens power using the post-operative corneal refractive power and an ocular axial length of the eye.

**6.** The method to determine the intraocular lens power according to claim 5, wherein the determination step divides the post-operative corneal shape into the corneal shape in the non-corrected region and a corneal shape in the corrected region so as to determine the non-corrected region.

**7.** The method to determine the intraocular lens power according to claim 5 further comprising a step of measuring the corneal shape by picking up an image of a measurement target projected onto the cornea of the eye, wherein the input step is for inputting data on the measured post-operative corneal shape.

**Patentansprüche**

**1.** Ophthalmologiegerät, das Eigenschaften einer Hornhaut eines Auges einer zu untersuchenden Person gewinnt, die verwendet werden, um eine Brechkraft einer intraokularen Linse zu bestimmen, die in das Auge eingesetzt werden soll, wobei das Gerät umfasst:

eine Eingabeeinheit (20), die Daten über eine Form der Hornhaut nach refraktiver Chirurgie eingibt; und eine Berechnungseinheit (20) mit einem Programm, das eine postoperative Hornhautbrechkraft auf der Grundlage der postoperativen Hornhautform berechnet, wobei
das Programm
einen nicht korrigierten Bereich der Hornhaut auf der Grundlage der postoperativen Hornhautform bestimmt;

**dadurch gekennzeichnet, dass** das Programm
eine präoperative Hornhautform in einem korrigierten Bereich durch Berechnen einer Näherungskurve aus einer Hornhautform in dem nicht korrigierten Bereich schätzt,
eine präoperative Hornhautbrechkraft auf der Grundlage der präoperativen Hornhautform berechnet;
eine Korrekturbrechkraft in der refraktiven Chirurgie auf der Grundlage der postoperativen Hornhautform und der präoperativen Hornhautform berechnet; und
eine postoperative Hornhautbrechkraft auf der Grundlage der präoperativen Hornhautbrechkraft und der Korrekturbrechkraft berechnet.

**2.** Ophthalmologiegerät nach Anspruch 1, wobei die Berechnungseinheit ferner ein Programm umfasst, das die Brechkraft der intraokularen Linse unter Verwendung der postoperativen Hornhautbrechkraft und einer axialen Länge des Augapfels des Auges bestimmt.

**3.** Ophthalmologiegerät nach Anspruch 1, wobei das Programm die postoperative Hornhautform in die Hornhautform in dem nicht korrigierten Bereich und eine Hornhautform in dem korrigierten Bereich unterteilt, um so den nicht korrigierten Bereich zu bestimmen.

**4.** Ophthalmologiegerät nach Anspruch 1, das ferner eine Hornhautform-Messvorrichtung (10) umfasst, die die Hornhautform durch Aufnehmen eines Bildes eines auf die Hornhaut des Auges projizierten Messziels misst, wobei die Eingabeeinheit Daten über die gemessene postoperative Form eingibt.

**5.** Verfahren zum Bestimmen einer Brechkraft einer intraokularen Linse, die in ein Auge einer zu untersuchenden Person eingesetzt werden soll, wobei das Verfahren die Schritte umfasst:

Eingeben von Daten über eine Form einer Hornhaut nach refraktiver Chirurgie;
Bestimmen eines nicht korrigierten Bereichs der Hornhaut auf der Grundlage der postoperativen Hornhautform;
Schätzen einer präoperativen Hornhautform in einem korrigierten Bereich durch Berechnen einer Näherungskurve aus einer Hornhautform in dem nicht korrigierten Bereich;
Berechnen einer präoperativen Hornhautbrechkraft auf der Grundlage der präoperativen Hornhautform;
Berechnen einer Korrekturbrechkraft in der refraktiven Chirurgie auf der Grundlage der postoperativen Hornhautform und der präoperativen Hornhautform; und
Berechnen einer postoperativen Hornhautbrechkraft auf der Grundlage der präoperativen Hornhautbrechkraft und der Korrekturbrechkraft; und

Bestimmen der Brechkraft der intraokularen Linse unter Verwendung der Brechkraft der postoperativen Hornhaut und einer axialen Länge des Augapfels des Auges.

**6.** Verfahren zum Bestimmen der Brechkraft der intraokularen Linse nach Anspruch 5, wobei der Bestimmungsschritt die postoperative Hornhautform in die Hornhautform in dem nicht korrigierten Bereich und eine Hornhautform in dem korrigierten Bereich unterteilt, um so den nicht korrigierten Bereich zu bestimmen.

**7.** Verfahren zum Bestimmen der Brechkraft der intraokularen Linse nach Anspruch 5, das ferner einen Schritt zum Messen der Hornhautform durch Aufnehmen eines Bildes eines auf die Hornhaut des Auges projizierten Messziels umfasst, wobei der Eingabeschritt der Eingabe von Daten über die gemessene postoperative Hornhautform dient.

**Revendications**

**1.** Appareil ophtalmique qui obtient des caractéristiques d'une cornée de l'oeil d'un patient examiné qui sont utilisées pour déterminer la puissance d'une lentille intraoculaire à injecter dans l'oeil, l'appareil comprenant :

une unité d'entrée (20) qui entre des données sur une forme de la cornée après une chirurgie réfractive ; et
une unité de calcul (20) possédant un programme qui calcule la puissance réfractive cornéenne postopératoire sur la base de la forme cornéenne postopératoire, dans lequel
le programme
détermine une région non corrigée de la cornée sur la base de la forme cornéenne postopératoire ; **caractérisé en ce que** le programme
évalue une forme cornéenne préopératoire dans une région corrigée en calculant une courbe approximative à partir d'une forme cornéenne dans la région non corrigée ;
calcule la puissance réfractive cornéenne préopératoire sur la base de la forme cornéenne préopératoire ;
calcule la puissance réfractive de correction lors de la chirurgie réfractive sur la base de la forme cornéenne postopératoire et de la forme cornéenne préopératoire ; et
calcule la puissance réfractive cornéenne postopératoire sur la base de la puissance réfractive cornéenne préopératoire et de la puissance réfractive de correction.

**2.** Appareil ophtalmique selon la revendication 1, dans lequel l'unité de calcul comprend en outre un programme qui détermine la puissance de lentille intraoculaire en utilisant la puissance réfractive cornéenne postopératoire et une longueur axiale oculaire de l'oeil.

**3.** Appareil ophtalmique selon la revendication 1, dans lequel le programme divise la forme cornéenne postopératoire en la forme cornéenne dans la région non corrigée et en une forme cornéenne dans la région corrigée de manière à déterminer la région non corrigée.

**4.** Appareil ophtalmique selon la revendication 1, comprenant en outre un appareil de mesure de forme cornéenne (10) qui mesure la forme cornéenne en prenant une image d'une cible de mesure projetée sur la cornée de l'oeil, dans lequel
l'unité d'entrée entre des données sur la forme cornéenne postopératoire mesurée.

**5.** Procédé pour déterminer la puissance d'une lentille intraoculaire à injecter dans l'oeil d'un patient examiné, le procédé comprenant les étapes consistant à :

entrer des données sur une forme d'une cornée après une chirurgie réfractive ;
déterminer une région non corrigée de la cornée sur la base de la forme cornéenne postopératoire ;
évaluer une forme cornéenne préopératoire dans une région corrigée en calculant une courbe approximative à partir d'une forme cornéenne dans la région non corrigée ;
calculer la puissance réfractive cornéenne préopératoire sur la base de la forme cornéenne préopératoire ;
calculer la puissance réfractive de correction lors de la chirurgie réfractive sur la base de la forme cornéenne postopératoire et de la forme cornéenne préopératoire ; et
calculer la puissance réfractive cornéenne postopératoire sur la base de la puissance réfractive cornéenne préopératoire et de la puissance réfractive de correction ; et
déterminer la puissance de lentille intraoculaire en utilisant la puissance réfractive cornéenne postopératoire et une longueur axiale oculaire de l'oeil.

6. Procédé pour déterminer la puissance de lentille intraoculaire selon la revendication 5, dans lequel l'étape de détermination divise la forme cornéenne postopératoire en la forme cornéenne dans la région non corrigée et en une forme cornéenne dans la région corrigée de manière à déterminer la région non corrigée.

7. Procédé pour déterminer la puissance de lentille intraoculaire selon la revendication 5, comprenant en outre une étape consistant à mesurer la forme cornéenne en prenant une image d'une cible de mesure projetée sur la cornée de l'oeil, dans lequel
l'étape d'entrée consiste à entrer des données sur la forme cornéenne postopératoire mesurée.

FIG. 1

EP 2 000 080 B1

```
                          ┌─────────────┐
                          │    START    │
                          └──────┬──────┘
                                 │
                    ┌────────────▼────────────┐
                    │   OBTAIN CORNEAL SHAPE   │
                    └────────────┬────────────┘
                                 │
            No          ╱────────▼────────╲
      ┌─────────────── ◄  HAVE BEEN CORRECTED? ►
      │                 ╲────────┬────────╱
      │                          │ Yes
      │               ┌──────────▼──────────────┐
      │               │ DETERMINE NON-CORRECTED REGION │
      │               └──────────┬──────────────┘
      │                          │
      │          ┌───────────────▼──────────────────┐
      │          │ CALCULATE PRE-OPERATIVE CORNEAL SHAPE │
      │          └───────────────┬──────────────────┘
      │                          │
      │          ┌───────────────▼──────────────────┐
      │          │ CALCULATE PRE-OPERATIVE CORNEAL REF. POWER │
      │          └───────────────┬──────────────────┘
┌─────▼──────┐                   │
│ CALCULATE  │   ┌───────────────▼──────────────────────────┐
│ CORNEAL    │   │ CALCULATE CORNEAL ABLATION AMOUNT DISTRIBUTION │
│ REF. POWER │   └───────────────┬──────────────────────────┘
└─────┬──────┘                   │
      │          ┌───────────────▼──────────────────┐
      │          │ CALCULATE CORRECTION REF. POWER   │
      │          └───────────────┬──────────────────┘
      │                          │
      │          ┌───────────────▼──────────────────────┐
      │          │ CALCULATE POST-OPERATIVE CORNEAL REF. POWER │
      │          └───────────────┬──────────────────────┘
      │                          │
      └──────────────────────────┤
                                 │
                    ┌────────────▼────────────┐
                    │ DISPLAY CALCULATION RESULT │
                    └────────────┬────────────┘
                                 │
                          ┌──────▼──────┐
                          │     END     │
                          └─────────────┘
```

# FIG. 2

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005030044 A
- US P20050225724 A
- JP 2005288176 A
- US P5500697 B
- JP H7124113 B
- US P6033075 B
- JP H11342152 B

**Non-patent literature cited in the description**

- **NAOYUKI MAEDA et al.** Corneal topographer and wavefront sensor. Medical View Co., Ltd, 10 October 2002